# EUROPEAN PATENT SPECIFICATION

(11) **EP 0 728 109 B1**
(45) Date of publication and mention of the grant of the patent: **19.04.2000**
(21) Application number: 95903104.8
(22) Date of filing: 01.11.1994
(51) Int. Cl.: B65D 55/08, B65D 41/40

(54) **ANAESTHETIC-AGENT CONTAINER COMPRISING A SINGLE-USE CLOSURE**
NARKOSEMITTELBEHÄLTER MIT EINWEGVERSCHLUSS
RECIPIENT D'AGENT ANESTHESIQUE COMPRENNANT UNE FERMETURE A USAGE UNIQUE

(30) Priority: 15.11.1993 IT MI932431
(43) Date of publication of application: 28.08.1996
(73) Proprietor: ABBOTT LABORATORIES, Abbott Park, Illinois 60064-3500 (US)
(72) Inventor: DE RUBEIS, Fabio, I-00042 Anzio (IT); GENGA, Rodolfo, I-00198 Rome (IT)
(74) Representative: Modiano, Guido, Dr.-Ing.
(86) International application number: US9412537
(87) International publication number: WO9513972

(56) References cited:
- DE-A- 2 624 753
- FR-A- 2 599 339
- GB-A- 5 776
- GB-A- 529 836
- IT-A- MI 932 316
- US-A- 3 592 351
- US-A- 4 241 841

## Description

The present invention relates to an anaesthetic agent container having a closure, which permits transfer of anaesthetic agent from a storage container to an administration system by means of one of a plurality of transfer methods which can be selected according to requirements.

Inhalable anaesthetics are typically volatile substances with relatively low boiling points and high vapor pressures. They can be flammable and explosive substances in both their liquid and vapor states. Such anaesthetics are typically dispensed in liquid form to an apparatus such as an anaesthetic vaporizer, which mixes the anaesthetic with carrier gases, normally oxygen and nitrous oxide. The mixture is supplied in gaseous form to the patient for inhalation. Since inhalation of the vapour by health care personnel using them can cause drowsiness, such anaesthetics must be safely stored in the hospital pharmacy and safely handled in operating rooms in order to minimize the risk of inhalation by health care personnel, as well as to minimize the risk of fire or explosion. Therefore, the anaesthetic agent should be stored and used in a way that will, ensure that there is no contamination of the atmosphere at all stages of handling during normal storage and surgical procedures.

Different types of closures have been developed for safely sealing anaesthetic agent containers. The type of closure used depends upon the method or device used for transferring the anaesthetic agent from the container to the vaporizer. Some closures do not permit such transfer of anaesthetic in a closed system that eliminates the escape of anaesthetic gases to the atmosphere. Typically, during setup procedures, a supply container of anaesthetic is opened in the operating room and either poured directly into a vaporizer or attached to a feed line leading to a vaporizer.

One such closure is constituted by a threaded aluminium cap incorporating a frangible sealing ring, known as a "Rolled On Pilfer Proof Cap" or R.O.P.P. cap. Such closures are widely used and are advantageous in that by unscrewing the cap in an opening direction, the frangible ring is broken and the cap easily and quickly unscrewed to give easy and rapid access to the contents of the container. R.O.P.P. caps are used for sealing some anaesthetic agent containers. Anaesthetic agents may be transferred from such containers by using a standard reusable I.S.O. filling device or by pouring the anaesthetic directly into the vaporizer reservoir. This permits very rapid charging of the vaporizer as may be necessary e.g., in the instance of an unscheduled emergency surgical intervention, but results in a period of time during which the open container of anaesthetic is exposed to the atmosphere. Accordingly, in order to avoid the risks of inhalation by health care personnel, fire or explosion, suitable precautions such as ensuring adequate ventilation, must be adopted when transferring anaesthetic agent from such containers sealed with R.O.P.P. caps to a vaporizer.

Closures have been required for use with devices which have been developed to permit the transfer of an anaesthetic agent from a container to a vaporizer. Some of these devices do not permit anaesthetic to be transferred from a container to a vaporizer in a closed system. U.S. Patent 5,144,991 discloses a filling device for an anaesthetic vaporizer with a temperature sensitive switching member. U.S. Patent 4,867,212 discloses a safety arrangement for filling and emptying an anaesthetic vaporizer. However, when using these devices, the supply container remains open to the atmosphere for a period of time while being connected to the vaporizer, therefore allowing anaesthetic to escape to the atmosphere.

Further devices were developed which eliminated the escape of anaesthetic gas to the atmosphere. Such devices have a vaporizer adaptor that engages with an anaesthetic vaporizer, and a closure adaptor which engages with a closure on the anaesthetic container. The closure is preferably connected to the supply container prior to use in the operating room. The container closure has a frangible seal adapted to be perforated by piercing means within the closure adaptor as the adaptor is engaged with the closure. Following perforation of the frangible seal of the closure by the piercing means of the adaptor, the closure adaptor and the closure remain held together in a snap fit, thereby permitting transport of anaesthetic from the supply container to the vaporizer in a system which remains closed to the atmosphere throughout assembly or disassembly procedures. One such system is known as the ABBOTT Security Lock Filling System, and is available from Abbott Laboratories, Abbott Park, Illinois 60064-3500, U.S.A.

Furthermore, the shape of the closure adaptor is frequently chosen to mate with a corresponding shape of closure. In this way, containers of anaesthetic may have closures of different shapes to avoid accidents whereby the container is hooked up to an incorrect vaporizer.

Although these developments have improved the safe handling of anaesthetic agents, other problems have arisen regarding manufacture and use of the available anaesthetic container closures.

Storage and inventory problems have arisen since containers having different types of closure but containing the same type of anaesthetic have to be stored in order to be able to take full advantage of the different methods of filling vaporizer reservoirs, according to requirements. Thus, for example, a hospital would typically store an anaesthetic agent in sealed containers having frangible aluminium R.O.P.P. caps, for pouring directly into a vaporizer reservoir or for use with standard reusable I.S.O. agent-specific filling devices. The same anaesthetic agent would typically also be manufactured and stored in a hospital pharmacy in sealed containers having closures comprising a frangible seal adapted to be perforated by piercing means within the closure adaptor of an anaesthetic transfer system, such as the above mentioned ABBOTT Security Lock Filling System.

Since the shapes of the closure adaptor of the various transfer devices in use are frequently chosen to mate with a corresponding shape of closure, the same anaesthetic may also have to be stored in containers of anaesthetic having closures of different shapes to mate with the different adaptors of the transfer devices in use in the hospital. This avoids accidents by preventing the container from being hooked up to an incorrect vaporizer, but complicates storage and inventory. In fact, the number of anaesthetic agents being stored at any one time is multiplied by the number of different types of container also required for use with the various anaesthetic transfer systems and adaptors in use with a number of different vaporizers.

Furthermore, in unscheduled emergency surgery, in a life-saving situation where immediacy of anaesthetic administration is of prime priority, valuable time can be lost while seeking the correct anaesthetic in the correct container for use with a specific vaporizer in what may be the only available operating room. The possible consequences of every second wasted in such an emergency situation are obvious.

Problems are also created for manufacturers of sealed containers filled with anaesthetic agents, since various production lines have to be set up for containers having different types of closure for each anaesthetic agent. Specifically, threaded neck glass bottles coupled to screw caps have to be provided for transferring anaesthetic agent to a vaporizer via direct pouring or via I.S.O. agent specific filling devices, such as the devices commonly known as standard Vapofill with "Safety Pin" vaporizer connector. On the other hand however, standard flanged mouth glass bottles have to be used with anaesthetic transfer devices such as the ABBOTT "Security Lock" filling system.

A container closure, particularly for anaesthetic containers is described in Italian Patent Application No. MI93A 002316, filed by the same applicant on October 29, 1993. Whilst such closure has been found to solve the above mentioned problems, it has also been found to be susceptible to improvement.

DE-A-26 24 753 teaches a removable closure for a pharmaceutical container. The closure includes an opening element having a cutting edge for piercing the bottom portion of a reservoir provided in the closure. The opening element is pushed downwards to rupture the bottom of reservoir to release a substance (e.g. a powder) contained therein for intermixing with a fluid provided in the pharmaceutical container.

US-A-3 592 351 discloses a closure for a barrel of draft beer having a sealing plug which extends substantially inwardly with respect to a top plate.

In particular, it is desirable to provide an improved anaesthetic container closure, attached to a container, which cannot be reused with another container upon being removed therefrom and which can be connected to a user device.

Accordingly, an aim of the invention is to provide a closure particularly for anaesthetic agent containers which overcomes the problems encountered in the known anaesthetic agent containers.

Within this aim, an object of the invention is to provide a closure particularly for anaesthetic agent containers which permits an anaesthetic to be either poured directly into a vaporizer reservoir or selectively used with either a standard reusable I.S.O. agent-specific filling device, or with a container-to-vaporizer transfer device such as the currently used ABBOTT "Security Lock" Filling System.

Another object of the invention is to provide a closure for anaesthetic agent containers which permits the anaesthetic to be selectively accessed as easily and as rapidly as could be achieved by using frangible aluminium "Rolled On Pilfer Proof" (R.O.P.P.) caps.

Another object of the invention is to provide a closure for anaesthetic agent containers which is selectively perforatable by piercing means within the closure adaptor of an anaesthetic transfer system.

A further object of the invention is to provide a closure for anaesthetic agent containers which can safely retain a closure adaptor of an anaesthetic transfer device in operative engagement therewith.

A still further object of the invention is to provide a closure which permits a single type of bottle to be employed for direct pouring or for use with I.S.O. agent specific filling devices or with a container-to-vaporizer transfer device of the type having a vaporizer adaptor and a closure adaptor that engages with the closure on the anaesthetic container, such as the ABBOTT "Security Lock" filling system.

A further object of the invention is to provide a closure for anaesthetic containers, which, upon being attached to one container, cannot be detached therefrom and reused with a different container.

With these and other objects in view, there is provided an anaesthetic agent container comprising a closure as defined in claim 1. Further features and advantages of the invention will become apparent from the following detailed description thereof, and the following drawing wherein:

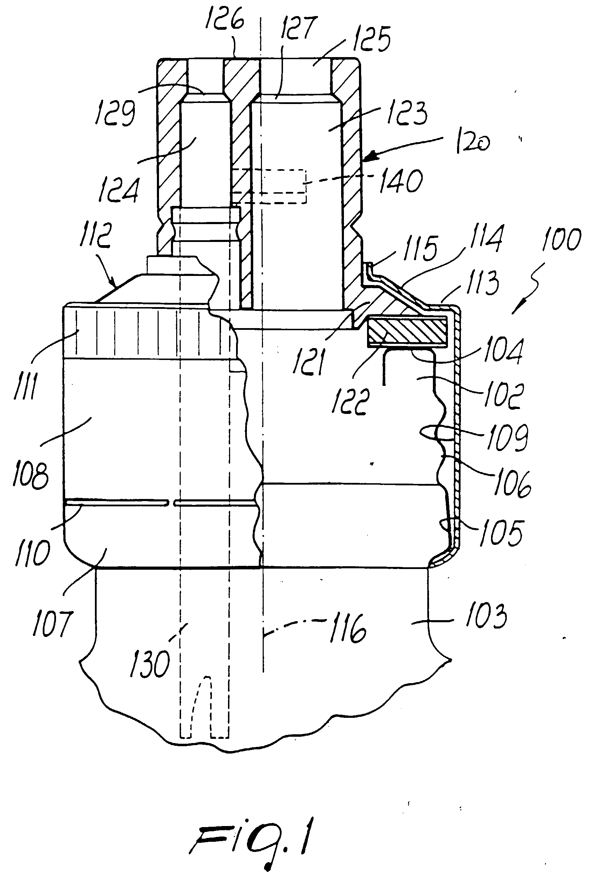

fig. 1 is a cross sectional view of the container closure according to the invention, attached to the threaded neck of an anaesthetic container.

With reference to the above described drawing figure, the closure according to the invention is generally indicated by the reference numeral 100. The closure is shown in engagement with the neck 102 of an anaesthetic container 103 which, in the illustrated example, is constituted by a standard threaded neck glass bottle 103. The neck 102 defines an upper edge 104 and an externally protruding annular rib 105. Screw threads 106 are formed on the outer surface of the neck 102 and extend axially between the annular rib 105 and the upper edge 104.

The closure 100 has a locking ring 107 which engages the externally protruding annular rib 105 of the container 103 and is connected to a collar 108. The collar 108 is connected to the ring 107 at a frangible annular band 110, and preferably has knurled or ribbed annular band 111 formed thereon for facilitating gripping by a user when applying a torque force for separating the collar 108 from the ring 107 by breaking the frangible annular band 110.

Above the portion of the collar 108 defining the knurled band 111, the collar has an annular flange 112 defining, in cross section, a first flange portion 113 connected to the annular band 111 and extending substantially perpendicularly with respect to the longitudinal axis 116 of the closure 100; a second flange portion 114 connected to the first flange portion 113 and extending on a plane which is inclined with respect to the longitudinal axis 116 of the closure 100; and a third flange portion 115 connected to the second flange portion 114 and extending parallel to the longitudinal axis 116 of the closure 100.

The closure 100 further comprises a stopper member 120 defining a bottom flange 121 having an external surface engaged by the annular flange 112 of the collar 108, and an internal surface in sealing engagement with the upper face of an annular sealing member or gasket 122. The gasket 122 is interposed between the bottom flange 121 and the upper edge 104 of the container 103. Engagement of the locking ring 107 with the externally protruding annular rib 105 of the container 103 maintains a compressive force, which is exerted through the collar 108 and flange 112, on the bottom flange 121 and on the gasket 122. Thus, the upper face of gasket 122 is in sealing abutment engagement with the lower internal surface of the bottom flange 121, while the lower face of the gasket 121 is in sealing abutment engagement with the upper edge 104 of the container 103.

A discharge passage 123 and a venting passage 124 extend through the stopper member 120, parallel to the longitudinal axis 116 of the closure 100. The discharge passage 123 defines a discharge opening 125 at the upper end 126 of the stopper member, and is occluded by a first pierceable membrane 127, located within the discharge passage 123, preferably at a location inwardly spaced from the discharge opening 125. Similarly, the venting passage 124 defines a venting opening 128 and is occluded by a second pierceable membrane 129, located within the venting passage 124, preferably at a location inwardly spaced from the venting opening 128. A one way check valve 130 can be press-fitted within the venting passage 124 if desired to ensure that no liquid can flow through it.

A locking means for connecting the closure to an anaesthetic transfer device may also be provided. Advantageously, the locking means comprise an outwardly projecting wedge 140, for engagement with a recessed edge formed on a transfer device.

The closure described so far is substantially identical to the closure described in Italian Patent Application No. MI93A 002316, filed by the same applicant on October 29, 1993. According to the present invention, the closure also comprises means for preventing reuse of the closure upon being detached from a container. Advantageously, the means for preventing reuse of the container comprise a planar face 109, defined on the internal face of the collar 108 facing the screw threads 106 formed on the neck 102 of the container 103. Thus there is no engagement between the collar 108 and the screw threads 106.

The closure 100 is securely and safely retained in a correct position of the container 103 by the engagement of the locking ring 107 with the protruding rib 105 of the container 103. The contents of the container may be transferred to a vaporizer either by connecting the discharge and venting passages 123, 124 to an anaesthetic transfer device, or by rupturing the frangible annular band 110 in order to completely remove the closure 100 from the container 103 and permit the contents to be poured directly into a vaporizer, as fully described in the above-mentioned Italian Patent Application No. MI93A 002316.

Upon removal of the closure 100 from the container 103 by rupturing the frangible annular band 110, the planar face 109 prevents the closure from being reused with another container by preventing engagement with the threaded neck of such container. Reuse of the closure according to the invention on successive container is thus prevented.

The closure member 100 may be applied to containers containing a specific type of anaesthetic, and the stopper member may be provided with a configuration for matching a transfer device for a vaporizer intended for use with such specific anaesthetic. BY providing the planar face 109, the closure 100 cannot be removed from one agent-specific container, and used on a different type of container. Thereby, it becomes impossible to transfer a specific type of anaesthetic to the wrong vaporizer.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Container containing an anaesthetic agent and comprising a closure (100) with a locking ring (107) connected to a collar (108) at a frangible annular portion (110) and means (109) for preventing reuse of said closure (100) upon being detached from said container (103),
wherein said closure (100) further comprises means (120-129) fixed to and extending outwardly from said collar (108) for selectively and detachably connecting said closure (100) to a user device.

2. The container according to claim 1, characterized in that said means (109) for preventing reuse of said closure (100) upon detachment from said container (103) comprises a planar surface (109) defined internally on said collar (108).

3. The container according to claims 1 and 2, characterized in that said planar surface (109) is an annular planar surface.

4. The container according to one or more of the preceding claims, characterized in that said means (120-129) for selectively and detachably connecting said closure (100) to a user device comprises a stopper member (120).

5. The container according to claim 4, characterized in that said stopper member (120) comprises a discharge passage (123) and a venting passage (124) which extend axially through said closure (100).

6. The container according to claim 5, characterized in that said discharge passage (123) is occluded by a first pierceable membrane (127) located within said discharge passage (123), and/ or in that said venting passage (124) is occluded by a second pierceable membrane (129) located within said venting passage (124).

7. The container according to claims 5 or 6, characterized in that a one way check valve (130) is press-fitted within said venting passage (124) to ensure that no liquid can flow through said venting passage (124).

8. The container according to one or more of claims 1-7, characterized in that said means (120-129) for selectively and detachably connecting said closure (100) includes a locking means (140) comprising an outwardly projecting wedge adapted for locking said closure (100) to an anaesthetic transfer device.

## Patentansprüche

1. Ein Narkosemittel enthaltender Behälter, der einen Verschluß (100) mit einem Verschlußring (107) umfaßt, der an einem zerbrechbaren ringförmigen Abschnitt (110) mit einer Manschette (108) verbunden wird, und ein Mittel (109) zum Verhindern der Wiederverwendung des Verschlusses (100) umfaßt, nachdem er vom Behälter (103) gelöst wurde,
worin der Verschluß (100) weiterhin Mittel (120-129) umfaßt, die an der Manschette (108) angebracht sind und sich nach außen davon erstrecken, um den Verschluß (100) selektiv und lösbar mit einer Benutzervorrichtung zu verbinden.

2. Der Behälter nach Anspruch 1, dadurch gekennzeichnet, daß das Mittel (109) zum Verhindern der Wiederverwendung des Verschlusses (100) beim Lösen vom Behälter (103) eine ebene Fläche (109) umfaßt, die innen an der Manschette (108) bestimmt ist.

3. Der Behälter nach den Ansprüchen 1 und 2, dadurch gekennzeichnet, daß die ebene Fläche (109) eine ringförmige ebene Fläche ist.

4. Der Behälter nach einem oder mehreren der vorherigen Ansprüche, dadurch gekennzeichnet, daß die Mittel (120-129) zur selektiven und lösbaren Verbindung des Verschlusses (100) mit einer Benutzervorrichtung ein Stopfenglied (120) umfassen.

5. Der Behälter nach Anspruch 4, dadurch gekennzeichnet, daß das Stopfenglied (120) einen Ablaßdurchlaß (123) und einen Lüftungsdurchlaß (124) umfaßt, die sich axial durch den Verschluß (100) erstrecken.

6. Der Behälter nach Anspruch 5, dadurch gekennzeichnet, daß der Ablaßdurchlaß (123) durch eine erste einstechbare Membran (127) verschlossen wird, die innerhalb des Ablaßdurchlasses (123) angeordnet ist, und/oder dadurch, daß der Lüftungsdurchlaß (124) durch eine zweite einstechbare Membran (129) verschlossen wird, die innerhalb des Lüftungsdurchlasses (124)angeordnet ist.

7. Der Behälter nach den Ansprüchen 5 oder 6, dadurch gekennzeichnet, daß ein Ein-Wege-Rückschlagventil (130) innerhalb des Lüftungsdurchlasses (124) preßgepaßt wird, um sicherzustellen, daß keine Flüssigkeit durch den Lüftungsdurchlaß (124) fließen kann.

8. Der Behälter nach einem oder mehreren der Ansprüche 1-7, dadurch gekennzeichnet, daß die Mittel (120-125) zur selektiven und lösbaren Verbindung des Verschlusses (100) ein Verschlußmittel (140) einschließen, das einen nach außen ragenden Keil umfaßt, der ausgebildet ist, um den Verschluß (100) an einer Narkosemittel-Überführungsvorrichtung zu verriegeln.

## Revendications

1. Récipient contenant un agent anesthésique et comportant un dispositif de fermeture (100) pourvu d'une bague de blocage (107) raccordée à un collet (108) situé au niveau d'une partie annulaire (110) qui peut être rompue, et des moyens (102) servant à empêcher une réutilisation dudit dispositif de fermeture (100) lorsqu'il est détaché dudit récipient (103),
dans lequel ledit dispositif de fermeture (100) comporte en outre des moyens (120-129) fixés à et s'étendant à l'extérieur dudit collet (108) pour raccorder d'une manière sélective et amovible ledit dispositif de fermeture (100) à un dispositif d'utilisateur.

2. Récipient selon la revendication 1, caractérisé en ce que lesdits moyens (109) servant à empêcher une réutilisation du dispositif de fermeture (100) lors de son détachement dudit récipient (103) comprennent une surface plane (109) définie intérieurement dans ledit collet (108).

3. Récipient selon les revendications 1 et 2, caractérisé en ce que ladite surface plane (109) est une surface plane annulaire.

4. Récipient selon l'une ou plusieurs des revendications précédentes, caractérisé en ce que lesdits moyens (120-129) pour raccorder d'une manière sélective et amovible ledit dispositif de fermeture (100) à un dispositif d'utilisateur comprennent un élément d'arrêt (120).

5. Récipient selon la revendication 4, caractérisé en ce que ledit élément d'arrêt (120) comprend un passage de refoulement (123) et un passage d'aération (124) qui s'étendent axialement à travers ledit dispositif de fermeture (100).

6. Récipient selon la revendication 5, caractérisé en ce que ledit passage de refoulement (123) est fermé par une première membrane (125) pouvant être perforée, qui est située dans ledit passage de refoulement (123), et/ou en ce que ledit passage d'aération (124) est fermé par une seconde membrane (129) pouvant être perforée, située dans ledit passage d'aération (124).

7. Récipient selon les revendications 5 ou 6, caractérisé en ce qu'une soupape unidirectionnelle antiretour (130) est montée à force dans ledit passage d'aération (124) de manière à garantir qu'aucun liquide ne peut circuler dans ledit passage d'aération (124).

8. Récipient selon une ou plusieurs des revendications 1-7, caractérisé en ce que lesdits moyens (120-129) pour le raccordement sélectif et amovible dudit dispositif de fermeture (100) comprennent des moyens de blocage (140) comprenant un coin, qui fait saillie extérieurement et est adapté pour verrouiller ledit dispositif de fermeture (100) sur un dispositif de transfert d'anesthésique.
